(19) **Europäisches Patentamt / European Patent Office / Office européen des brevets**

(11) **EP 3 745 416 A1**

(12) # EUROPEAN PATENT APPLICATION

(43) Date of publication:
**02.12.2020 Bulletin 2020/49**

(51) Int Cl.:
**G16H 50/70** (2018.01)    **G16H 10/60** (2018.01)
**G16H 70/20** (2018.01)

(21) Application number: **19177330.8**

(22) Date of filing: **29.05.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **JACOBS, Igor**
  **5656 AE Eindhoven (NL)**
• **VERBURG, Pepijn**
  **5656 AE Eindhoven (NL)**

• **PLUYTER, Jon Ragnar**
  **5656 AE Eindhoven (NL)**
• **LANGEREIS, Sander**
  **5656 AE Eindhoven (NL)**
• **WILLIAMS, Sharon**
  **5656 AE Eindhoven (NL)**
• **HRISTOV, Zoran**
  **5656 AE Eindhoven (NL)**
• **NIE, Hongchao**
  **5656 AE Eindhoven (NL)**
• **LOOS, Marc**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **CLINICAL DECISION SUPPORT**

(57)    Presented are concepts for generating a subject-specific user interface for Clinical Decision Support. Once such concept comprises obtaining clinical reference information regarding established clinical knowledge, and also obtaining subject-specific clinical information regarding a subject. Clinical context information which describes a relevance or importance of at least one of a plurality of different clinical standards is determined. Based on the clinical reference information, the subject-specific clinical information and the clinical context information, a subset of the subject-specific clinical information relevant to a clinical decision is determined. At least one of: a user-interface component; and a user-interface element is defined based on the subset of the subject-specific clinical information.

EP 3 745 416 A1

FIG. 1

**Description**

FIELD OF THE INVENTION

This invention relates to the field of Clinical Decision Support and more particularly to user interfaces for Clinical Decision Support.

BACKGROUND OF THE INVENTION

Technological advances are leading to an increase in the amount and complexity of data collected in a variety of settings and processes. Such data may not be useful unless it can be integrated, filtered and interpreted to obtain actionable insights. This requires dedicated decision support systems and computer algorithms that can transform the data to actionable insights. An exemplary domain where the use of such systems is becoming increasingly relevant is healthcare.

High-quality care delivery requires optimal decisions on care management to be made along a subject's care trajectory. However, both healthcare professionals and subjects (e.g. patients) can be faced with complex clinical decisions (e.g. due to an increasing volume and complexity of available data). This may, for example, be due to rapidly expanding disease knowledge and technological advances that not only include novel or more advanced diagnostic tools and therapies, but also an increased availability of data sources and healthcare digitalization.

Although healthcare information technology is increasingly adopted and transforming modern healthcare delivery, healthcare information systems do not filter and transform the available data to a level that is easily understandable and actionable in the care process. Consequently, clinical decision support (CDS) systems are becoming an integral component of healthcare information technology.

CDS systems can significantly improve the clinical, financial and workflow aspects of care, both at an individual subject level and at a population health management level. However, effective adoption and implementation of CDS systems has proven to be difficult. Key challenges faced in increase the effectiveness of CDS systems include:

improving the user interface;
summarizing subject-specific information;
prioritizing and filtering recommendations;
combining recommendations for subject with comorbidities; and
using free text information to drive clinical decision support.

SUMMARY OF THE INVENTION

The invention aims to at least partly fulfil the aforementioned needs. To this end, the invention provides devices, systems and methods as defined in the independent claims. The dependent claims provide advantageous embodiments.

There is provided a method for generating a subject-specific user interface for Clinical Decision Support, the method comprising:

obtaining clinical reference information regarding established clinical knowledge, the established clinical knowledge relating to a plurality of different clinical standards;
obtaining subject-specific clinical information regarding a subject;
determining, based on the subject-specific clinical information, clinical context information describing a relevance or importance of at least one of the plurality of different clinical standards;
based on the clinical reference information, the subject-specific clinical information and the clinical context information, determining a subset of the subject-specific clinical information, the subset comprising subject-specific clinical information relevant to a clinical decision; and
defining at least one of: a user-interface component; and a user-interface element based on the subset of the subject-specific clinical information.

Proposed embodiments provide concepts for generating flexible and/or adaptive User Interfaces (UIs). Such generated UIs may provide for personalized (e.g. subject-specific) clinical decision support within a problem space that is so large that it does not allow handcrafting a UI design for each problem separately. Embodiments may be used to automatically generate personalized UIs which contain features specific to a particular subject (e.g. patient) and context.

Embodiments may help to provide the correct data, in the correct format and timing/hierarchy in a clinical workflow and medical context. This may enable a medical professional to analyze a subject-specific case based on a UI that is optimized to show the relevant data for that subject.

By way of example, proposed methods may utilize a knowledge inference component (e.g. inference engine) and a clinical pathway model to extract out of the full set of subject-specific clinical information: (i) a relevant subset of the subject-specific clinical information and (ii) potentially use this relevant subset to generate personalized recommendations for a clinical problem at hand, e.g., diagnostic test results relevant to treatment selection decision.

Proposed concepts may provide a UI engine that is configured to automatically generate (rather than handcraft) a UI that is optimized to show the relevant subset of the subject-specific clinical information and/or personalized recommendations for a given problem at hand in a given clinical context (e.g., user, physical space) using a set of UI components. By way of example, in a proposed embodiment, the UI engine selects the optimal UI components and then composes them into a dedicated UI for that particular subject.

Embodiments may employ clinical pathway models as part of the UI engine, and may optionally employ other knowledge bases such as hospital-specific clinical rules, or artificial intelligence on local subject data. This may facilitate an improved presentation of decision support outcome or problems/issues to the user, improving usability and facilitating better decision-making. Improved effectiveness may lead to improved outcomes, reduced cost, better workflow and improved user experience.

Proposed embodiments may address the challenge of clinical decision support systems presenting the right data, at the right time, in the right format. For instance, this may be done by automatically generating a user interface based on the importance of information elements relative to the context and to each other, according to a variety of factors. The interface elements and components of the user interface may be automatically given a certain appearance and relevance, depending on the relevance of information. Further, the components may not only be adaptive/responsive to themselves, but may also flexibly adapt to the other components of the interface. This may ensure that the importance of a set of components can be emphasized rather than individual data points. It may also ensure that the hierarchy of information is maintained and that relations or discrepancies between key information elements remain evident. In this way, embodiments may define a set of user-interface components in a clinically relevant way.

Reference to a User Interface (UI) should be taken as reference to the environment (e.g. two-dimensional screen representation) through which a user and a decision support system interacts or exchanges information, comprising a set of UI components orchestrated in a predefined hierarchy and order.

A UI component comprises an integrated representation of a relevant subset of subject-specific clinical information that is filtered out of a larger dataset (e.g. based on clinical reference information and clinical context information). This may be built up from multiple UI elements or other UI components.

A UI element comprises a singular representation (e.g. visual or textual) of specific clinical information.

A proposed embodiment may infer, from knowledge bases (e.g. containing clinical reference information regarding established clinical knowledge) and subject-specific clinical information, which are the relevant data elements, what is the information hierarchy with respect to information relevance and what are important relations/associations for making decisions for a subject in a certain clinical context.

Embodiments may employ an output component that consumes information from a decision-support system and infers it with factors that affect optimal user-interface configuration. These factors may, for example, comprise: the content, context, data format, setting & physical space, role & profile, technology & media and historic decisions.

The output component may automatically render a user-interface for a decision support system, in which the presence, relevance and appearance of the UI elements and components are triggered by the factors such as those listed in the preceding paragraph. Such factors can affect the content, composition, appearance and behavior of the UI elements and components. For instance, the user interface engine may assign subject-specific clinical information, and user interface elements and components in which the information reside, certain weights depending on their relevance. It may then perform a calibration of all subject-specific clinical information and user-interface elements and components to resolve their appearance relative to each other. In this way, the user-interface elements and components can be flexibly and dynamically selected from a library in which they are available in various configurations, and their layouts then adapted to a proportion-based grid following content-first, responsive design principles. The UI elements and components may be responsive to their content and other UI elements and components.

The output component may adjust a "region-of-interest" to focus on (i.e., emphasize) a set of relevant features. Also, features of past, present, and (possibly) future may be displayed. This can lead to an improved presentation of decision-support outcome or problems/issues to a user/viewer, improving usability and facilitating better decision-making.

Algorithms or models employed by embodiments may comprise simple linear models or statistical models, and/or employ artificial intelligence, depending on the number of factors taken into account and the complexity of the aggregated data.

Proposed embodiments may thus help a user (such as a medical practitioner, doctor, surgeon, technician, etc.) to be presented with relevant or important subject-specific clinical information in a manner which takes account of a clinical context. Subject-specific clinical information identified in consideration of a clinical context may enable a user to focus on particular clinical information of interest or relevance, thus reducing complexity or confusion that may otherwise be caused by presenting excessive and/or irrelevant information.

Embodiments may increase a value of clinical information by employing contextual information to identify information in a manner that accounts for a specific (e.g. selected, chosen or current) clinical context. Embodiments may increase the clinical value of subject-specific clinical information by employing contextual information rather than relying on technical expertise of a user.

Obtaining clinical reference information may comprise obtaining clinical reference information from at least one of: a knowledge-based system comprising a clinical knowledge base with domain knowledge, rules and associations; and a non-knowledge-based system comprising a learning component configured to generate clinical knowledge. Two main types of CDS systems are knowledge-based and non-knowledge based systems.

Knowledge-based systems typically comprise three main components: (i) The knowledge base with domain knowledge, rules and associations; (ii) The inference engine, which does the reasoning based on the combination of knowledge with the subject's data; and (iii) the user interface, the means with which the user interacts with the decision support system and via which the outcome of the decision support system is presented to the user. In such a system, knowledge bases can comprise various forms of clinical reference information, including clinical guidelines or pathways, expert advices, or online data such as research evidence or other literature. Clinical guidelines are a set of recommendations for optimal management of subjects with specific conditions, based on research evidence and clinical practice experience. A subject's care pathway describes the sequential steps in a subject's multidisciplinary care process.

Non-knowledge based systems typically use artificial intelligence or statistics to generate knowledge by learning from clinical experience or finding patterns in clinical data, for example local subject cohort data (both clinical and operational). Similarly to knowledge-based systems, they also typically comprise an inference engine and user interface.

In some embodiments, the subject-specific clinical information may comprise at least one of: subject data describing at least one physiological parameter or characteristic of the subject; clinical data describing at least one medical parameter or characteristic of the subject; and operational data describing at least one clinical test or procedure undertaken by the subject. Subject-specific clinical information may thus include a variety of data types, including subject information (e.g. medical history, comorbidities, etc.), test results (e.g. medical imaging, pathology and molecular pathology, laboratory tests, etc.), received treatments and treatment outcomes, psychosocial data, operational data (e.g. process quality, data age, etc.), etc.

The clinical reference information may comprise a clinical pathway model. Determining clinical context information may then comprise: analyzing the clinical pathway model of the subject-specific clinical information to identify a current location of the subject in a clinical pathway; identifying one or more of the plurality of different clinical standards as being relevant or importance based on the identified current location of the subject in a clinical pathway; determining a relevance or importance of the identified one or more clinical standards; and determining clinical context information describing the determined relevance or importance of the identified one or more clinical standards.

In an embodiment, determining a subset of the subject-specific clinical information may comprise: associating weighting values to the subject-specific clinical information based on the clinical reference information and the clinical context information; and identifying a subset of the subject-specific clinical information based on the weighting values associated with the subject-specific clinical information.

Further, associating weighting values to the subject-specific clinical information may comprise: processing the clinical reference information and the clinical context information with a heuristic algorithm to determine weighting values for the subject-specific clinical information.

In some embodiments, defining at least one of: a user-interface component; and a user-interface element based on the subset of the subject-specific clinical information comprises: identifying at least one data type of the subset of the subject-specific clinical information based on the weighting values associated with the subject-specific clinical information; and determining a user-interface component or user-interface element based on the identified data type. By way of example, this may comprise identifying a user-interface component or user-interface element from a set of available user-interface components and user-interface elements, wherein the identified user-interface component or user-interface element is configured to show data of the identified data type. For instance, determining a user-interface component or user-interface element may comprise: associating component weighting values to the at least one data type; and identifying a user-interface component or user-interface element from a set of available user-interface components and user-interface elements based on the component weighting values associated with the at least one data type.

Where the set of available user-interface components and user-interface elements does not contain a user-interface component or user-interface element that is suitable for showing data of the identified at least one data type, a new user-interface component or user-interface element for showing data of the identified data type. In this way, embodiments may be thought of as including a process of resolving the most appropriate user-interface component or user-interface elements for an identified data type. For instance, where the identified subset of the subject-specific clinical information indicates that a specific form of data needs to be conveyed to a user, embodiments may resolve this with reference to available user-interface components or user-interface elements that are each designed to convey particular forms of data. An optimal user-interface component or user-interface element for the subject-specific clinical information may thus be identified (or defined if no suitable components/elements are identified).

Accordingly, in an exemplary embodiment, defining at least one of: a user-interface component; and a user-interface element may comprise: selecting a user-interface component or user-interface element from a library of user interface components and elements.

Defining at least one of: a user-interface component; and a user-interface element may comprise: determining a value of a property of the user-interface component or element based on at least one of: a preference associated with the subject; a subject-specific setting associated with the subject; an historical value associated with a previous clinical decision for the subject; and a user input signal. For example, the value of a property of the user-interface component or element may be automatically generated while taking account of various factors and/or inputs.

Some embodiments may further comprise: generating a user-interface control signal for controlling the display of a user-interface, wherein the user-interface control signal comprises information descriptive of the defined user-interface component and/or user-interface element. Such an output signal may comprise a control signal for controlling the display of a user-interface component and/or user-interface element. Only subject-specific clinical information identified as being relevant or important with respect to a clinical context or decision may be displayed, thereby preventing irrelevant and/or extraneous information from being provided to a user.

According to another aspect, there is provided a computer program product for generating a subject-specific user interface for CDS, wherein the computer program product comprises a computer-readable storage medium having computer-readable program code embodied therewith, the computer-readable program code configured to perform all of the steps of a proposed embodiment.

Thus, there is also provided a computer system comprising: a computer program product according to proposed embodiment; and one or more processors adapted to perform a method according to a propose concept by execution of the computer-readable program code of said computer program product.

According to another aspect of the invention, there is provided a system for generating a subject-specific user interface for Clinical Decision Support, the system comprising: an interface component configured to obtain clinical reference information regarding established clinical knowledge, the established clinical knowledge relating to a plurality of different clinical standards, and to obtain subject-specific clinical information regarding a subject; an inference component configured to determine, based on the subject-specific clinical information, clinical context information describing a relevance or importance of at least one of the plurality of different clinical standards; an analysis component configured to, based on the clinical reference information, the subject-specific clinical information and the clinical context information, determining a subset of the subject-specific clinical information, the subset comprising subject-specific clinical information relevant to a clinical decision; and an output component configured to define at least one of: a user-interface component; and a user-interface element based on the subset of the subject-specific clinical information.

mation.

The system may be remotely located from a display, and a definition of a user-interface component and/or a user-interface element may thus be communicated via a communication link. In this way, a user (such as a medical professional) may have an appropriately arranged system that can receive content/information at a location remotely located from the system for generating a subject-specific user interface for CDS. Embodiments may therefore enable a user to remotely retrieve and review subject-specific clinical information using a local system (which may, for example, comprise a portable display device, such as a laptop, tablet computer, mobile phone, PDA, etc.).

The system may further comprise: a server device comprising the system for generating a subject-specific user interface; and a client device comprising a user-interface display. Dedicated data processing means may therefore be employed for the purpose of generating a subject-specific user interface, thus reducing processing requirements or capabilities of other components or devices of the system.

The system may further comprise a client device, wherein the client device comprises the analysis component, an output component and a display unit. In other words, a user (such as a doctor or medical professional) may have an appropriately arranged client device (such as a laptop, tablet computer, mobile phone, PDA, etc.) which processes received data in order to define at least one of: a user-interface component; and a user-interface element and generate a display control signal.

Thus, it will be understood that processing capabilities may therefore be distributed throughout the system in different ways according to predetermined constraints and/or availability of processing resources.

These and other aspects of the invention will be apparent from and elucidated with reference to the embodiment(s) described hereinafter.

BRIEF DESCRIPTION OF THE DRAWINGS

Examples in accordance with aspects of the invention will now be described in detail with reference to the accompanying schematic drawings, in which:

Figure 1 is a simplified block diagram of a system according to an exemplary embodiment;
Figures 2A-2C illustrate a block diagram of an exemplary implementation of the analysis component of Figure 1;
Figure 3A-3C depict simplified examples of user interfaces associated with an exemplary use case of an embodiment;
Figure 4 is a schematic illustration of a proposed approach which provides different UIs;
Figure 5 is a conceptual illustration of a care path driven UI according to a proposed embodiment;
Figures 6A-6C depict first to third instances of an exemplary UI generated according to a proposed embodiment, respectively; and

Figure 7 is a simplified block diagram of a computer within which one or more parts of an embodiment may be employed.

DETAILED DESCRIPTION OF THE EMBODIMENTS

Proposed is a concept for enabling the provision of subject-specific clinical information that is relevant or important for a specific clinical context. Embodiments may provide a UI based on the relevance or importance of information for a clinical context and/or clinical decision.

Such proposals may thus facilitate simple and intuitive navigation and identification of subject-specific clinical information in consideration of a clinical context (e.g. clinical setting, state or environment), for example by enabling pertinent or relevant information to be visualized and/or navigated with regard to a clinical context. Improved CDS may therefore be provided by proposed embodiments.

Embodiments of the present invention are therefore directed toward enabling the presentation of subject-specific clinical information with reference to a clinical context so as to facilitate or enhance a CDS process. Further, embodiments may be aimed at enabling the display or visualization of relevant or important subject-specific clinical information with simplicity, accuracy and clinical context while providing enough detail so that a clinical assessment or decision is facilitated or supported (preferably with increased efficiency and/or reliability). Embodiments may therefore assist in clinical support assessment, medical diagnosis and/or support planning for example.

Illustrative embodiments may be utilized in many different types of clinical, medical or subject-related environments, such as a hospital, doctor's office, ward, care home, person's home, etc. In order to provide a context for the description of elements and functionality of the illustrative embodiments, the Figures are provided hereafter as examples of how aspects of the illustrative embodiments may be implemented. It should therefore be appreciated that the Figures are only examples and are not intended to assert or imply any limitation with regard to the environments, systems or methods in which aspects or embodiments of the present invention may be implemented.

It is proposed that various factors are important for effective communication of a decision support outcome to a user, and it is preferable to account for such factor in a user interface for CDS. Examples of such factors include:

**The content:**

Different disease types require different content (e.g. subject history, medical imaging, or pathology results). Broader application of the decision support system requires a scalable solution.

**The context:**

For example, with which purpose is the system being used at that particular moment in time. If the subject is in the primary diagnostic phase, the user interface may put more emphasis on that, showing the strength of the diagnostic evidence, discrepancy in information and missing tests. After the subject would have an established diagnosis and the treatment is to be selected, more emphasis may be placed on subject fitness and likelihood of various treatments to be successful. During clinical surveillance, an up-to-date health status may be shown in the context of the disease history, possibly augmented with indications of current issues and health risks.

**The data format:**

For example, structured vs. non-structured information: structured data is organized, coded and may have known relations with other data elements. This enables that structured data can for example be placed in dedicated fields of the user interface. Furthermore, relations and associations between structured data elements can be shown. In addition, graphical representations can be used for structured data elements or relations between them to make information more glanceable. However, when data would be text-based, the user interface would need to be adapted to make the text readable and the right amount of text should be displayed to remain comprehensible.

**The setting / physical space:**

For example, a multidisciplinary team meeting, consultation, etc. In a multidisciplinary team meeting the user interface should support group discussions in which decisions have to be made within very limited time. The evidence from multidisciplinary evaluation should be comprehensible for the whole team. In a consultation, a clinician may need easy access to more detailed information and possibilities to switch between an expert view and a subject view.

**The role / profile:**

The user type (e.g. clinical discipline or subject) and user profile affect the optimal configuration. For example, during case review a clinical specialist may need to see more detail of his own discipline in the user interface, placed in the context of information from other discipline. Besides, a clinician may need specialized information, whereas a subject may need information that is easier understandable.

**The technology / media:**

Each viewing platform (e.g. monitor, computer screen, tablet, smart phone, etc.) requires an optimal configuration to make the information readable.

**Historic decisions and accompanying user interfaces:**

The system may learn from decisions in previous settings and the efficacy of decision-making at these moments. Also, user preferences set in different moments in time are taken into account.

Each of these factors may affect the relevance of specific data or combination of data (in general and relative to each other) and thereby their preferred appearance and relevance on the user interface. The inventors propose a system that generates flexible and adaptive UIs that are driven by the CDS systems, factors such as described above and subject-specific data. The relevant information and required presentation format may be automatically extracted and a system may then adaptively render the UI interface depending on the above factors.

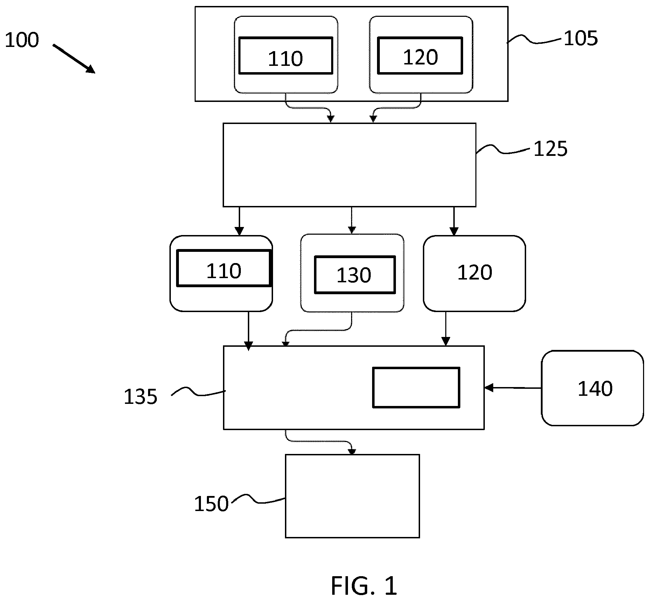

Referring now to Figure 1, there is depicted a simplified block diagram of a system according to an exemplary embodiment. The CDS system 100 comprises an interface component 105 that is configured to obtain knowledge-based clinical reference information 110 and subject-specific clinical information 120.

By way of example, the clinical reference information 110 may be knowledge-based: a rule-base, e.g. specifying if-this-then-that statements based on clinical guidelines, clinical pathways, expert advice, online data, etc. In other embodiments, the clinical reference information 110 may be non-knowledge-based: generated knowledge, e.g. from application of data statistics or artificial intelligence to learn from local cohort data, global data, etc.

The subject-specific clinical information 120 may, for example, comprise: (i) Patient Data: demographics, socio-economic, medical history, etc.; (ii) Clinical Data: patient fitness, complaints, laboratory data, diagnostic data, treatment data, etc.; and/or (iii) Operational Data: date of exams, duration between exams, image and test quality, etc.

An inference component 125 is configured to determine, based on the subject-specific clinical information 120, clinical context information 130 describing a relevance or importance of at least one of the plurality of different clinical standards. By way of example, the inference component of this embodiment employs an inference Engine which enriches the clinical reference information 110 and the subject-specific clinical information 120 with clinical context information 130 by inferring what clinical reference information 110 is applicable for the subject-specific clinical information 120. For example, the inference engine may infer where in a clinical pathway the subject is, and this may be based on what examinations are completed. The position in the clinical pathway may indicate, for instance, which decision needs to be made and what data is important for this decision.

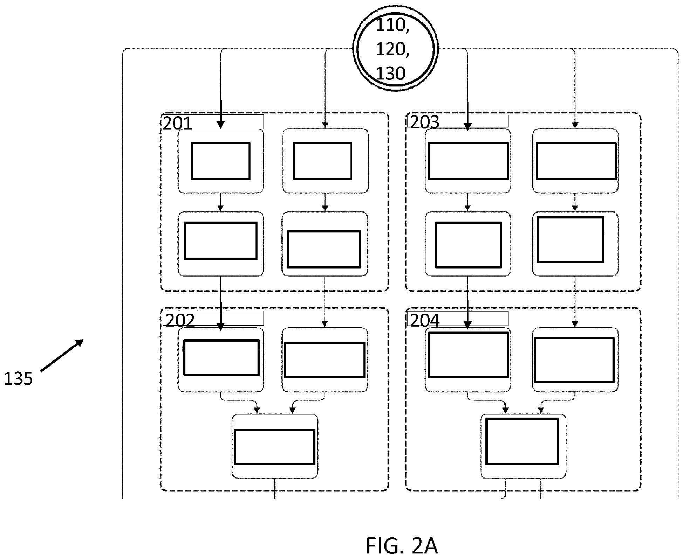

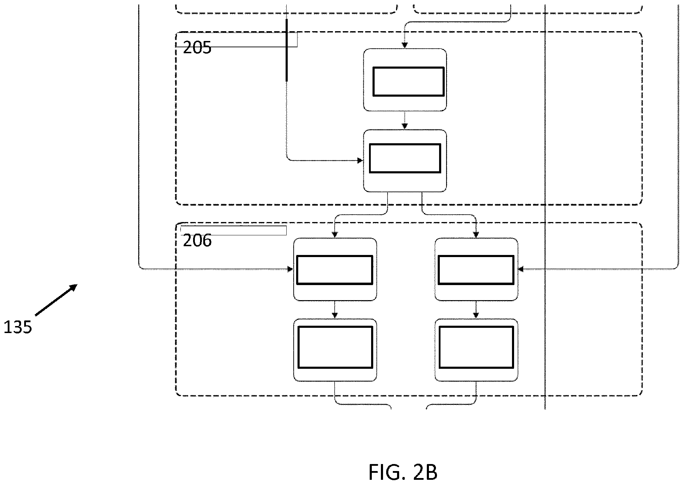

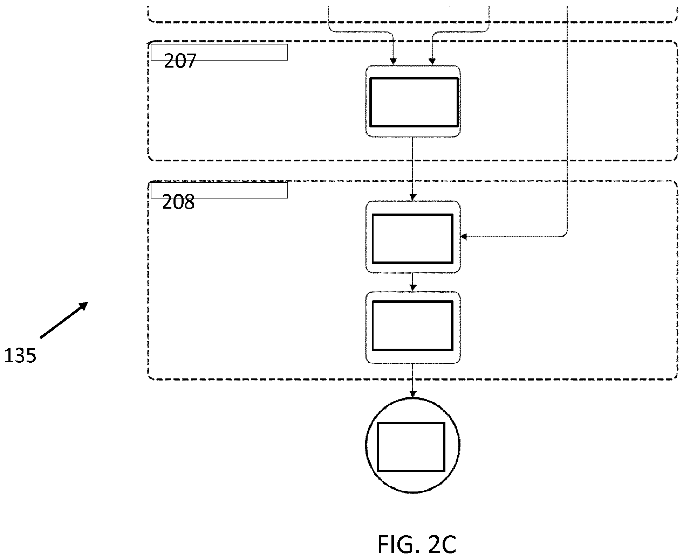

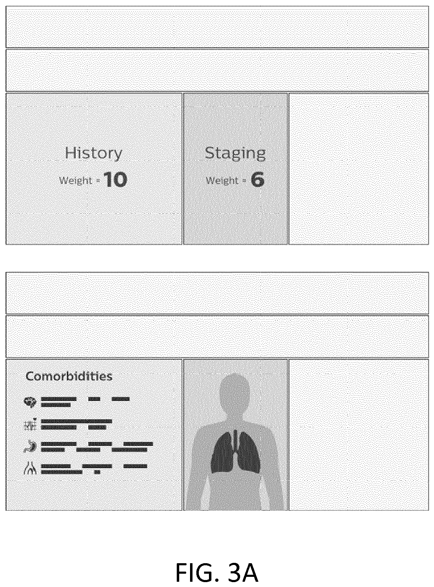

An analysis component 135 is configured to, based on the clinical reference information 110, the subject-specific clinical information 120 and the clinical context information 130, determine a subset of the subject-specific clinical information, the subset comprising subject-specific clinical information relevant to a clinical decision. Thus, the data input to the analysis component comprises the complete set of clinical reference information 110, the subject-specific clinical information 120 and the clinical context information 130. The analysis component of this embodiment comprises a UI engine that takes into account a variety of drivers that influence UI efficacy. The detailed functionalities of the analysis component 135 can be found below as described for Figures 2A-2B, and 3A-3C.

In this example, the UI engine of the analysis component 135 draws upon information from a UI library 140.

The UI library 140, for example, includes a library of UI elements, components and behavioral properties. Each element/component etc. is available in multiple configurations or appearances and with various degrees of detail and emphasis, e.g.: different sizes, different graphical representations for e.g. summarized and extended views, different highlighting methods, to e.g. stress novelty of data or discrepancy in data.

The UI library 140, also includes a UI framework containing general definitions, e.g.: a grid system on which the different elements and components can be mapped - The grid system is a determined number of columns of variable width fitted on the different screen sizes in which UI layouts are specified to fit the different components; visual language as the creation and study of visual representations to using graphic elements design principles to successfully communicate data; a set of rules for how the components and elements render for optimal understanding of data.

Further, the UI library 140 of this example comprises information relating to UI settings, such as preference settings. These may be pre-set, depending on e.g. user profiles. Also, there may be a certain calibration period, in which a user interacts with the automatically generated personalized UIs and makes adjustments in UI layouts and styling based on preferences, which can be stored and retrieved in the user's personal settings and from which the system can learn (e.g. utilizing hanging protocols). The may also be a library of historic settings resulting from previous cases that may be applicable to the current case.

Finally, the system 100 comprises an output component 150 configured to define at least one of: a user-interface component; and a user-interface element based on the subset of the subject-specific clinical information determined by the analysis component 135. The output component 150, for example, provides an integrated specification of UI components and elements in a grid-based layout, rendered on specified technical platforms. In this way, the output component is configured to output a representation of the clinical information determined by the analysis component 135.

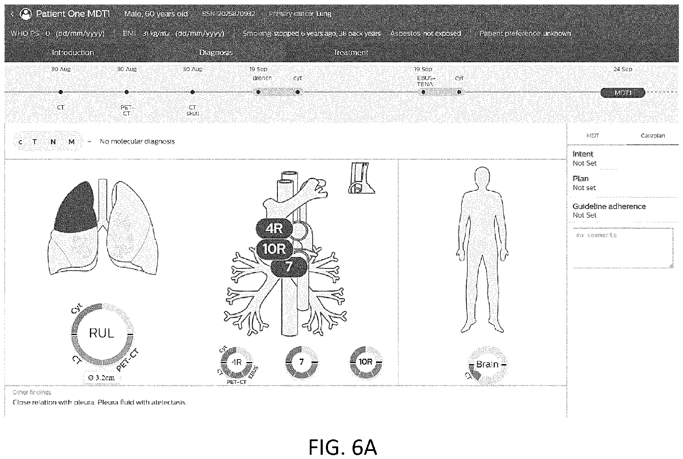

As detailed above, a UI element comprises a singular representation (e.g. visual or textual) of specific clinical information. By way of example, the graphical representation of a set of lungs in Figure 6A is an example of a UI element.

A UI component, on the other hand, comprises an integrated representation of a relevant subset of subject-specific clinical information that is filtered out of a larger dataset. This may be built up from multiple UI elements or other UI components. The combined representation of the bronchus and accompanying alphanumeric characters representing clinical information for the subject in the center portion of Figure 6A is an example of a UI component.

Referring now to Figures 2A-2C the analysis component 135 (i.e. UI engine) of Figure 1 is depicted in more detail.

The analysis component 135 drives data weights and UI components, calibrates and resolves them, and adjusts their visual appearance and behavior to render a flexible and dynamic UI. In summary, the engine does this by:

 identifying what data types are important;
 identifying which UI components are of interest;
 looking-up what data types are present in each of the UI components;
 calculating the importance for each UI component; and
 applying visual properties depending on the relative importance between UI components.

The analysis component 135 accomplishes this using the various component/entities described below and illustrated in Figure 2A-2C. To aid understanding and description of these, pseudo-code is used below to describe exemplary algorithms. Such algorithms can range from simple linear mathematical formulas (e.g. the operators used in the formulas below serve as an example), to more complex statistical models or machine learning implementations.

The components/entities in the analysis component comprise:

Data Weight Drivers 201

These specify which (types of) data (e.g. results of medical imaging, comorbidities, etc.) are relevant based on the clinical reference information 110, the subject-specific clinical information 120, and the clinical context information 130. Multiple Data Weight Drivers are activated depending on system configuration, each reacting to different subsets of the information. A Data Weight Driver 201 results in a list of weights for each data type. Weights are expressed as numerical values. It is possible to have multiple weights per data type (i.e. lists or matrices of weights) influencing different visual properties. For example, weight X is about visual size and weight Y is about visual data density. The translation from Data Input to weights per data type is a free-format rule base. One can choose a simple if-this-then-that rule engine or employ more complex statistical models or machine learning implementations.

Examples of Data Weight Drivers include:

(A) A 'Clinical Pathway Data Weight Driver'

This is where clinical guidelines or pathways are used as a carrier (knowledge-based decision support). These describe, for a certain disease type, what would be the recommended steps at different moments of the care path, e.g. using a decision tree. Required data elements that are important to make specific clinical decisions are (to a certain extent) predictable, which can drive the weight of certain data types. All weights combined create a unique profile of data relevance. Each node in the decision tree has a set of 'mutators' (e.g. simple mathematical transformations) specifying how the weight of each data type required to make the clinical decision corresponding to that node is adjusted when that node is active. For example, the relevance of diagnostic evidence from CT at node X is increased by 1 and multiplied by 2. At a subsequent decision node Y, a diagnosis is already established and relevance decreases: it is only relevant as contextual information and the weight is only increased by 1.

The required data to make specific decisions can also be used prospectively, looking forward at what are the options according to the guidelines/pathways and what the required data to make the decisions with respect to these options (and their pro's and con's).

A heuristic algorithm can be employed to determine initial weights in the pathway driver, by using the pathway knowledge model to define relevance of data in longitudinal overviews.

From this point of view, the decision points are connected by flows along the pathway. A pathway has multiple decision points, each of which has a finite number of local data variables as direct inputs. Besides, there may be variables that serve as indirect input for the decision point; e.g. they can be of influence, but are not listed in the care path model as recommended tests for that specific decision point.

Formula definitions:

The heuristic algorithm assigns weights to data according to their relevance for the decision point of interest. These weights contribute to the final calculation formula for 'Clinical Pathway Data Weight Driver' described below. It follows the principle that at a specific decision point, all weights should add up to 1. However, the way in which different data are weighed can vary across different decision points.

In the proposed definition, the care pathway model has D number of decision points. At a certain decision point, there potentially is a total number of $N_v$ input variables (both direct and indirect ones), as e.g. described by the pathway model.

Direct input data are defined as local variables ($lv_1$, $lv_2$, ..., $lv_n$), where the total number of local variables is $N_{lv}$

Two examples of indirect input data can be: Variables that have significantly changed (i.e. changed variables; $cv_1$, $cv_2$, ..., $cv_{Ncv}$) with respect to previous values of these variables, where the total number of changed variables is $N_{cv}$

Variables that are discordant (i.e. discordant variables; $dv_1$, $dv_2$, ..., $dv_{Ndv}$) with respect to previous values of these variables, where the number of discordant variables is $N_{dv}$.

Remaining variables are defined as rv, where the number of remaining variables is $N_{rv}$, where:

$$N_v = N_{lv} + N_{cv} + N_{dv} + N_{rv}$$

$$\forall\, x, y \in \{\, lv,\, cv,\, dv,\, rv \,\}, \, x \neq y: x \cap y = \varnothing$$

$$lv \cup cv \cup dv \cup rv = v$$

One can assign a fraction f for the indirect input variables to represent the influence that these data have on the current decision point.

Data input for the decision points:

Direct data input for current decision point: $(1-f)/N_{lv}$
Indirect data input for the current decision point: $f/(N_{cv}+N_{dv})$

Note that $N_v$ and $N_{lv}$ can be calculated directly from the pathway model, where $N_{cv}$ and $N_{dv}$ can only be determined during runtime, specific to the subject.

Then these weights are further taken in consideration amongst other data weight drivers and resolvers.

(B) A 'Meeting Data Weight Driver'

This is where the phase of the discussion determines what data is more relevant. For example, during a subject introduction in a multidisciplinary tumor board meeting, focus is on information related to a subject's medical history and high-level care path. Detailed information on diagnostic evidence and fitness for treatment is only relevant in a later phase of the discussion. Weights are therefore adjusted accordingly.

(C) A 'Role Data Weight Driver'

This is where the role / medical specialism of the user determines what data is more relevant. For example, data related to radiology has a bigger focus when in the 'radiologist role', e.g. during case preparation or case review from a role-perspective rather than a multidisciplinary perspective.

(D) A 'Subject Data Weight Driver'

This adjusts weights based on the content of the medical files of the subject. For example, if a CT scan is outdated, it should not influence the decision-making process to a full extent.

The total number of data types $T_i$ is $M$ and the set of all data types is $\mathbb{T} \equiv \{T_i\}_{i=1...M}$. Associated with each data type is a set of weights that influence the system (e.g. one can influence visual size assigned to the data type, another can change its visual emphasis). There is a total of N different weight types and the values for data type T are collected in a vector $\boldsymbol{w}_T$

$$\boldsymbol{w}_T = \begin{pmatrix} w_T^1 \\ w_T^2 \\ \dots \\ w_T^N \end{pmatrix}$$

where $w_T^i$ is the value of the weight of weight type $i$ for data type $T$. A data type may not use all weight types: the ones that it does not use can be set to 0 in the vector $\boldsymbol{w}_T$.

The weight vectors for all data types are collected in a matrix $W \in \mathbb{R}^{NxM}$:

$$W = (\boldsymbol{w}_{T_1}, \boldsymbol{w}_{T_1}, ..., \boldsymbol{w}_{T_M})$$

Each Data Weight Driver $D$ consists of a transformation $f_D : \mathbb{R}^{NxM} \to \mathbb{R}^{NxM}$ on W resulting in a matrix $W_D \in \mathbb{R}^{NxM}$ with numeric weights for each data type:

$$W_D = f_D(W)$$

$W_D^{i,j}$ denotes the element in the $i$th row and $j$th column of $W_D$ and it is the weight value of weight type $i$ for data type $T_j$ in the Data Weight Driver $D$.

There are L Data Weight Drivers $D_i$ active in the system.

Data Weight Resolver (202)

The Data Weight Resolver R combines all the independent lists of each Data Weight Driver 201 into one weight matrix $W_R \in \mathbb{R}^{NxM}$. From this matrix, it is known how relevant each Data Type is relative to others and this may be considered to identify the subset of the subject-specific clinical information.

The transformation $f_R : \mathbb{R}^{NxMxL} \to \mathbb{R}^{NxM}$ determines the Weights per Data Type of all Data Weight Drivers combined:

$$W_R = f_R(D_1, D_2, ..., D_L)$$

$W_R^{i,j}$ denotes the element in the $i$th row and $j$th column of $W_R$ and it is the weight value of weight type $i$ for data type $T_j$ in the Data Weight Resolver $R$.

It is noted that there are different choices for the exact transformation $f_R$. One can think of a simple mechanism adding up or calculating the average of all Weights grouped per Data Types and Weight Types. The operator can be chosen through a system configuration or via a learning system. Also, the Weights per Data Type from the Data Weight Drivers 201 can be multiplied with a constant: 'Data Weight Driver Multiplier' (M). This allows for contextual calibration between different Data Weight Drivers 201. The output from the Data Weight Resolver 202 may thus be thought of as identifying a relevant subset of the subject-specific clinical information (taking account of the clinical reference information, subject-specific clinical information and the clinical context information).

Component Drivers (203)

These specify what the composition should be of the UI components C. The Data Weight Drivers 201 are only aware of what data types are of increased relevance, but are not able to determine how they exactly relate to each other. For example, during a multidisciplinary tumor board meeting, the flow of past, present and future in data should be represented from left-to-right for better glanceability, following already established user mental models. An ordered list of components is defined in each Component Driver.

Another problem solved by Component Drivers 203 is the fact that a specific data type can exist in multiple UI components. Simply stating what data types are relevant would cause all of these UI components to be increased in relevance. Component Drivers give context to the UI and result in a set of applicable ordered UI components.

An example of a Component Driver 203 is a 'Meeting Component Driver' that selects the UI components based on where in the discussion the meeting is. For example, a conclusive phase would result in an additional component where the next step in the treatment plan can be noted.

There are P UI Components $C_i$ and the set of all UI Components is $\mathbb{C} \equiv \{C_i\}_{i=1...P}$. The UI Components for Component Driver $i$ can be described as an ordered subset $O_i$ of $\mathbb{C}$ :

$$O_i = \left( C_{i_1}, C_{i_2}, C_{i_{N_i}} \right)$$

where $N_i \leq P$ is the number of components of $i$ and $C_{i_j} \in \mathbb{C}$ for $j = 1, ..., N_i$.

There are Q Component Drivers $O_i$ active in the system.

Component Resolver (204)

Here, all the Component Drivers 203 are combined to create one ordered list $O$ of UI components that fits the current context (for example from old to new data when one needs to see the progression through time). The total of Q Component Drivers are combined through algorithm $f_O$:

$$O = f_O(O_1, O_2, ..., O_Q)$$

Function $f_O$ determines all the unique Component entries and orders them according to the current context. This is part of the business logic of the system.

Component Weight Resolver (205)

From the specification in the UI Library 140, it is known which data types exist in which UI components. It is also known what weights each data type has from the Data Weight Resolver 202. By combining data types per UI component and weights per data type it is possible to create a composite describing weights per UI component. This results in a metric defining what the relevance is of each UI component. This resolver is also able to see discordances between relevant data types and relevant UI components. For example: a data type is very relevant according to its weights, but there is no UI component selected to show this data. The resolver is able to 'mediate' and decide to render an additional UI component containing this crucial data type. The properties of the UI component may thus be configured according to properties (e.g. weights of different types) of the embedded data. For instance, a user-interface component or user-interface element may be identified (and/or defined) from a set of available user-interface components and user-interface elements based on component weighting values associated with the relevant data types.

First, for each UI component C an ordered Specification List $S_C \subset \mathbb{T}$ is fetched that contains all data types T in the component.

Three important elements are now known: (1) all Data Types and their Weights, (2) all Components in a specific order and (3) The Data Types that exist in each Component. The weight $W_{C_i,j}$ of the weight type $j$ for component $C_i$ is determined by a mapping $f_{C_i,j} \colon \mathbb{R}^{N_{C_i}} \to \mathbb{R}$:

$$W_{C_i,j} = f_{C_i,j}(W_R^{j,k_1}, W_R^{j,k_2}, \ldots W_R^{j,k_{N_{C_i}}})$$

where $k_i$ is the index of the data type at position $i$ in the ordered list $S_C$ (which is thus $T_{k_i}$) and $N_{C_i} \equiv |S_{C_i}|$.

Appearance Drivers (206)

These translate the abstract numerical weights (W) of each component (C) from the Component Weight Resolver to visual properties of a screen. For example, the clinical pathway mainly influences the 'column weight', defining how many columns (in the layout grid) a UI component gets relative to others. Another type of weight is 'depth weight', where the behavior expresses itself by determining which data type has the highest 'depth weight' and slowly enlarges and moves the content forward to occlude the other data and change focus. Emergent behavior appears when an UI component has multiple weights attached that visually play together. Each Appearance Driver may have contained functionality influencing specific visual properties for this to work properly.

Appearance Resolver (207)

Checks which visual properties are present on the UI components and calculates the merged visual properties for each UI component. When multiple Appearance Driver influence the same visual property, the driver with the highest priority according to an ordered list as part of the system configuration is chosen.

User Interface Renderer (208)

This combines all the UI components with the new visual properties, and finally rendering the User Interface.

This cycle of the User Interface Engine repeats itself when a user interaction adjusts properties of either Data Input, Data Weight Drivers, Component Drivers or Appearance Drivers. Changes propagate all the way down from weights per data type, to weights per UI component, to visual properties to, finally, the User Interface.

To provide the above description of the analysis component 135 with more context, a simple example based on a subject use case will now be described below, wherein the subject has a suspected cancer. Exemplary resulting (and greatly simplified) UIs associated with this exemplary use case are shown in Figures 3A-3C. The calculations, the effects that the weights exert on the UI components, and the simplified graphic representations are provided to serve as an example of how the analysis component 135 could function.

Pre-treatment phase:

In this example, the subject is in the primary diagnosis and staging phase of the care path. The diagnostic tests have been performed, but the definitive diagnosis and stage have not been established yet. The subject is discussed in a multidisciplinary team meeting.

From the subject's clinical information, the system identifies the position of the subject in the care path or decision tree (e.g. based on performed tests, the fact that the diagnosis and stage are not reported in the subject record yet, or the fact that the subject is selected for a first multidisciplinary tumor board meeting). The test results may be queried from the healthcare information system. Natural language processing may be used prior to the meeting to extract the test results from the plain text reports.

Based on the clinical guidelines, the decisions that need to be made at this moment in time are: (i) what is the diagnosis and stage? and (ii) what is the recommended treatment? (e.g. is the subject operable or inoperable?).

Relevant information for the diagnosis and stage decision is: an overview of which tests have been performed; and an overview of the results of these tests

Relevant information for treatment selection is: is the subject operable or inoperable, e.g. based on general fitness, organ function, comorbidities; and what are the available treatment options?

Before the treatment can be selected, clinicians first need to decide on the definitive diagnosis and stage. The UI should represent this information through the right set of UI components in a comprehensive way, easily glanceable for in a multidisciplinary team meeting. The system knows that it is being used in this setting since e.g. a certain viewing mode is selected. If data are structured/coded, a graphical representation can be used to make information glanceable; e.g. a graphic of the organ, with an indication of the specific location. A data model is used to describe structure and hierarchy of information. Data are coded with e.g. SNOMED CT, LOINC and ICD-10.

The Component Driver 203 defines that for this subject with e.g. suspected lung cancer, part of the diagnosis and staging component of the UI are graphic representations of the lungs and regional lymph node stations (and distant organs if there is a risk for distant metastases), shown from left to right. It can also determine that the structured test results can be shown with a schematic representation of the test result.

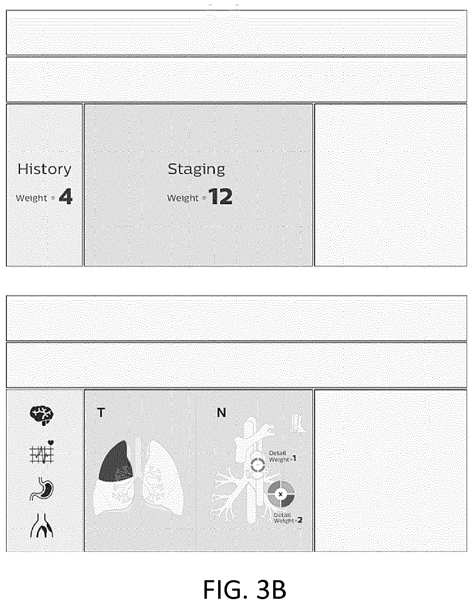

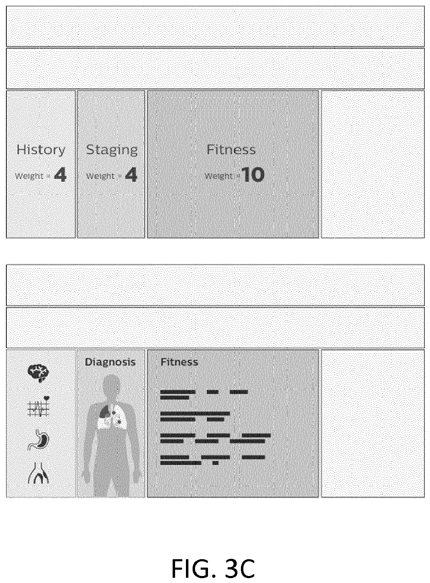

For the multidisciplinary meeting it is preferable that the UI components are logically ordered from past, to present, to future. This means respectively starting with the UI component subject history, then the staging and finally fitness tests for future treatment selection. The subject history example and fitness example are shown in Figure 3A and 3C, respectively, but are not further detailed in the text. The staging example is shown in Figure 3B.

The top diagram of Figure 3A shows a schematic representation of the UI, illustrating the influence of data weight on screen occupation. The bottom diagram of Figure 3A shows a simplified graphic representation of the UI, generated according to the relative weights in the schematic representation. In the subject introduction phase of the meeting, the relative weights of subject history data and staging data are 10 and 6. Hence, the UI is rendered to present comorbidities more prominent than staging information. Selecting from the library of components, comorbidities are shown in extended view with corresponding size and appearance. The staging information is shown in summarized view, using a graphic representation for the organ with the abnormality (e.g. lungs in this case).

The top figure of Figure 3B shows a schematic representation of the UI, illustrating the influence of data weight on screen occupation. The bottom diagram of Figure 3B shows a simplified graphic representation of the UI, generated according to the relative weights in the schematic representation. In the diagnosis and staging phase of the meeting, the relevance of the subject history is lower than information on staging from diagnostic tests. The UI is therefore rendered to present comorbidities more prominent than staging information, with relative weights of subject history data and staging data of 4 and 12, respectively. Selecting from the library of components, comorbidities are shown in summarized view (e.g. only icons). The staging information is shown in extended view, showing a graphic representation of the lungs (left) and regional lymph node stations (right). It uses circular charts to represent the diagnostic results, in which each section represents the result from a diagnostic modality. Colors may be used to indicate a level of suspicion for malignancy. For regional lymph node station "x" the test results are discordant. Therefore, it has an increased detail weight compared to the other lymph node station, resulting in a more prominent graphic representation.

The top diagram of Figure 3C shows a schematic representation of the UI, illustrating the influence of data weight on screen occupation. The bottom diagram of Figure 3C shows a simplified graphic representation of the UI, generated according to the relative weights in the schematic representation. In the treatment selection phase of the meeting, the relevance of the subject history and staging information is lower than subject fitness. The UI is therefore rendered to present fitness more prominent than comorbidities and staging information, with relative weights of subject history data, staging data and fitness data of 4, 4 and 10, respectively. Selecting from the library of components, comorbidities are shown in summarized view (e.g. only icons). The staging information is also shown in summarized view; only a summary of the diagnosis. Subject fitness is shown in extended view.

The data types living within the UI components appear, grow or have other visual appearances depending on the weights provided by the Data Weight Drivers 201. This means certain UI elements that were positioned in a certain way will not appear at all if they prove not to be relevant by the Data Weight Drivers 201 and with that free up space for other UI elements.

In this example, there is one Component Driver 203 active. Therefore, the ordering of the UI components stays the same as specified in the Multidisciplinary Meeting Component Driver.

Continuing first with the Data Weight Drivers 201, the current phase of the meeting is the diagnosis and staging phase, where it is important to know the strength of the diagnostic evidence. The subject's disease history has already been explained during the introductory phase of the meeting. Therefore, the Meeting Data Weight Driver 201 gives the comorbidities data type a limited column weight of 4 (see Figure 3B).

The clinical pathway model (knowledge-based) describes the set of recommended/required information for making that decision, e.g. based on the presumptive stage that has been documented in the CT report, pre-treatment evaluation should comprise test A, B, C and D. The Clinical Pathway Data Weight Driver gives the results of test A, B, C and D column weight of 2.

If tests are missing, or outcomes of these tests are discordant with each other (e.g. within a branch of the decision tree), that may be highlighted in the UI to reduce the risk of under- or over-staging.

Adherence to the guidelines can be inferred from the subject-specific data and the pathway model. A connection is made between the UI environment and pathway model via an API. In this example, the results of test B and D are discordant with respect to findings at body location X, e.g. a regional lymph node station (e.g. test B indicates high suspicion for malignancy, whereas test D indicates no suspicion). This finding needs additional attention during the multidisciplinary meeting. Therefore, the Subject Data Weight Driver 201 gives a column weight of 1 to test B and D. An additional detail weight of 1 is set on the data type of body location X living within test B and D (See Figure 3B).

The Subject Data Weight Driver 201 is configured to be of greater influence in comparison with the other Data Weight Drivers, because subject specific data is of bigger importance for this system configuration. Its weights are multiplied by 2.

The Data Weight Resolver 202 merges the weights from the different Data Weight Driver 201 vectors to one vector per data type and taking their multipliers into account:

> Comorbidities: column weight = 4
> Test A: column weight = 2
> Test B: column weight = 2 + 1 * 2 = 4
> Test C: column weight = 2
> Test D: column weight = 2 + 1 * 2 = 4
> Body location X: detail weight = 1 * 2 = 2

As an intermediate step, the Component Weight Resolver 205 collects the data types per UI components that is requested to render. For the sake of keeping the upcoming lists simple, mainly the subject history and staging UI component will be mentioned. The subject history UI component contains both the comorbidities and previous cancers data types. The staging component contains results from test A, B, C and D.

The Component Weight Resolver 205 then calculates all the weights per UI component based on the data types they contain (from step 11) and the weights per data type (from step 10). For example, the UI components will now have the following weights (note that this list does not include UI components living inside these top-level UI components):

> Subject history: column weight = 4
> Staging: column weight = 2 + 4 + 2 + 4 = 12 ; and detail weight = 2

The merged weights are then used to determine the visual appearances per component. Starting with the Column Appearance Driver 206, which reacts to the column weights. Normally there are also several default UI components visible with a fixed size, such as general subject information, footers, etc. Columns (of a 24 column grid system) are divided appropriately trough the total column weight of 4 + 12 = 16:

> Subject history: 24/ 16 * 4 = 6 columns
> Staging: 24/ 16 * 12 = 18 columns

The Detail Appearance Driver 206 reacts to the detail weights. Body location X for test B and D get an increased zoom-level to show a particular detail in the visual anatomical representation.

The visual properties of all the Appearance Drivers 206 are merged into one. Conflicting visual properties are resolved using a priority specification of which Appearance Driver is more important.

Finally, the merged visual properties are applied to the ordered UI components and rendered as User Interface Output:
Subject history is shown with 6 columns.

A graphical representation of the lungs, lymph nodes and metastases, with an visualization of the extent of disease for staging, occupying 18 columns.

Circular pie-charts that show the diagnostic results, in which test A-D are represented because of their column weight.

The discordance between test B and D is highlighted for body location X (a regional lymph node station), due to their higher detail weight.

After the diagnosis and stage have been established, the multidisciplinary discussion transitions to treatment selection. This moment can be known to the system from a certain user interactions with the interface.

Now, only a summarized version of the diagnostic results (e.g. only the tumor stage, cell type) is required; a Meeting Data Weight Driver 201 assigns less weight to the diagnosis and staging data. The Data Weight Drivers 201 assign more weight to data elements related to subject fitness, such as comorbidities and organ function. The Component Drivers 203 define a summarized version of the diagnosis and staging component and a panel to show fitness for treatment.

Data types are again collected for the different UI components, which get a certain weight depending on their content. The UI focuses now on fitness for treatment and treatment options, in context of the diagnosis and stage.

A treatment is selected and the subject transitions further in his/her care path.

As the subject further progresses through his/her care path, subsequent episodes of care will follow.

Post-treatment phase:

The subject undergoes treatment (e.g. surgery) and is discussed at a post-surgery MDT meeting. The flow starts from the top of Figure 2A again.

Based on the clinical guidelines, the decisions that needs to be made at this moment in time are: i) what are the findings at surgery (e.g. what is the post-operative stage, what are the treatment margins) and ii) what is the recommended follow-up (e.g. adjuvant treatment)

The system knows that now the information importance and hierarchy has changed, based on:

> the prior information was part of an earlier branch in the decision tree;
> there has been already a pretreatment MDT meeting; and
> a definitive diagnosis and stage have been reported in the health information system, and the system contains e.g. scheduled appointments or reports that indicate that e.g. surgery has been performed.

Therefore, the focus is placed on the post-surgical findings. The pre-treatment evaluation information becomes contextual information.

The appearance of the post-surgical findings should be the more detailed version, with a focus on post-surgical stage, resection margins, high-risk factors, etc.

The appearance of the pre-treatment findings should be the summarized version, only providing the context and highlighting any discrepancies.

The analysis component 135 regulates this again by assigning weights to data and data types, selecting the right components and their configuration from the library, merging and resolving everything together to flexibly and dynamically render a user-interface that contains the key information to support decision-making for that particular subject in that specific context.

Such an example is extendable to every moment in the care path, where the guidelines/pathways define the decision points, the required information to make the decision, principles/guidelines for evaluation, etc. The previous steps in the care path (inferred from the route the subject walked through the care path) provide information on what is contextual information. Subject-specific data in the context of the guidelines/care path can thus be highlighted.

Intra-branch missing or discordant information can be given a certain weight, flag, or association, impacting the user interface.

For longitudinal viewing, the system shows the up-to-date status, in the context of previous assessments. Longitudinally missing or discordant information can again be given a certain weight, flag, or association, impacting the user interface. In summary, different use cases (e.g. different patients) can have different disease types, or combinations of diseases, for which they follow a certain care path. Along these different care paths, various decision moments may occur, for which dedicated decision support is needed. A UI needs to effectively communicate the decision support output for that specific case at that moment, and an optimal UI may be dependent on this (as well as a number of other (variable) factors). It is proposed that combination of this information can automatically drive the UI configuration, such that the decision support can be optimally delivered. The proposed system may be configured to automatically generates these personalized UIs, which contain features dedicated to that a specific subject and context.

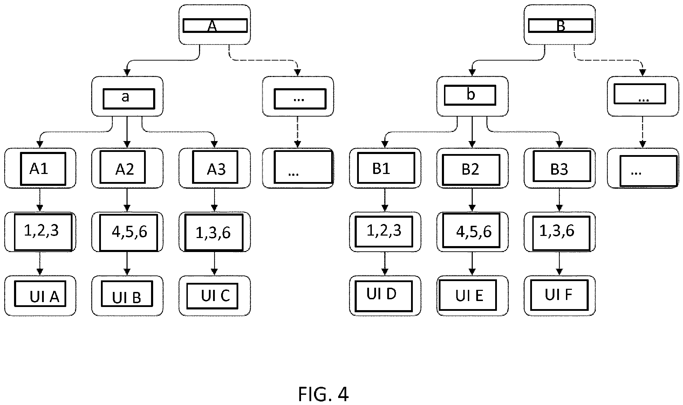

By way of illustration, Figure 4 is a schematic illustration of the concept that the proposed system can provide different UIs using, depending on use case and context (e.g. phase in the care path). For example, for a subject case A, a left hand path is taken if disease or condition "a" is present/diagnosed. This left-hand path has three decision points, A1, A2 and A3. For the first decision A1, three data weight drivers "1,2,3" are employed and result in a first UI component/element configuration "UI A". For the second decision A2, three respective data weight drivers "4,5,6" are employed and result in a second UI component/element configuration "UI B". For the third decision A3, three respective data weight drivers "1,3,6" are employed and result in a third UI component/element configuration "UI C".

Similarly, for a subject case B, a left hand path is taken if disease or condition "b" is present/diagnosed. This left-hand path has three decision points, B1, B2 and B3. For the first decision B1, three data weight drivers "1,2,3" are employed and result in a fourth UI component/element configuration "UI D". For the second decision B2, three respective data weight drivers "4,5,6" are employed and result in a fifth UI component/element configuration "UI E". For the third decision B3, three respective data weight drivers "1,3,6" are employed and result in a sixth UI component/element configuration "UI F".

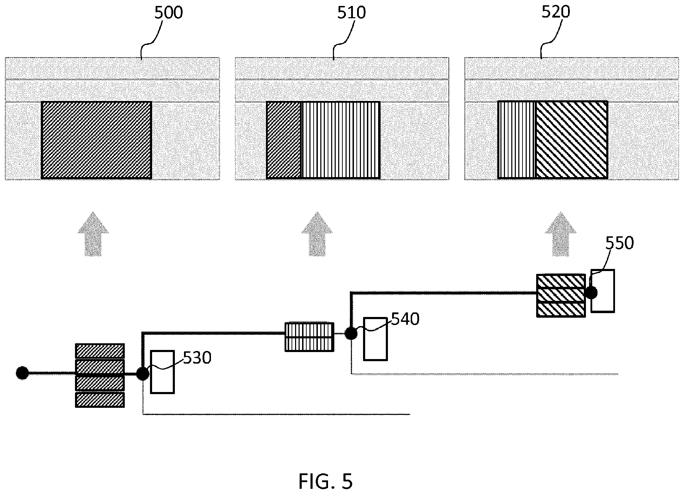

By way of further illustration, this is conceptually illustrated in Figure 5 based on an example of a clinical pathway.

Figure 5 is a conceptual illustration of a care path driven UI. The UI is dependent on use case and context (e.g. phase in the care path). The three UI dashboards 500, 510, 520 represent the UI at different moments in the care path. The bottom half of the illustration of Figure 5 shows the care path, wherein the numbered bullets 530, 540, 550 represent the different moments of decision-making and the pattern-filled bars recommended clinical tests according to the guidelines that are input for decision-making at that point.

At the first moment 530, information from a first set of test is driving the UI 500.

At the second moment 540, information from a second set of test has the highest importance for making the next decision, but information from the first set of tests is still important contextual information. Their respective relative relevance drives the UI 510.

At the third moment 550 in time, information from a third set of tests has the highest importance for decision-making and is most prominent in the UI. Information from the second set of tests is important contextual information, but information from the first set of tests is not. The respective relative importance of information from the third and second sets of tests drives the UI and the UI is not affected anymore by information from first set of tests.

By way of yet further illustration, two main steps of a proposed embodiment may be summarized as follows:

Step 1: - Example 1:

1) Patient data is captured in a clinical data model describing a broad set of potentially-relevant patient data. The patient data weight driver assigns weights to all patient data so as to indicate the respective relevance of the various patient data.

2) Clinical pathway conditions are captured in a clinical pathway model describing a potentially-relevant set of data elements. The clinical pathway data weight driver assigns weights to the data elements described by the clinical pathway conditions.

3) Patient data captured in the clinical data model is matched to the clinical pathway model, based on an identified position of the patient in the clinical pathway. This defines the relevant subset of subject-specific information, which based on matching the patient profile to the pathway conditions can automatically adapt to the position of the patient in the pathway.

Step 1: -Example 2:

1) Patient data is captured in a clinical data model describing a broad set of potentially-relevant patient data. The patient data weight driver assigns weights to all patient data so as to indicate the respective relevance of the various patient data.

2) The meeting weight driver assigns weights to all data relevant in the diagnosis phase of the MDT meeting. This defines the subset of subject-specific information, that can be extracted from the broader set of relevant patient data.

Step 2:

The data weight resolver combines the result of Step 1 above in a calibrated weight assigned to the subset of subject-specific information by the subject data weight driver, the clinical pathway data weight driver and the meeting weight driver.

Referring now to Figures 6A-6C, there are depicted first to third instances of an exemplary UI generated according to a proposed embodiment. More specifically, Figures 6A-6C are screenshots (i.e. captured images of a displayed UI) of a prototype UI for a first patient case. The UI comprises a dashboard which is configured to a patient overview at respective time/moment in the patient's care path. The dashboard is therefore adapted to provide a patient overview at different moments in a patient care path.

At each time/moment in the care path, the patient case was discussed in a multidisciplinary team meeting (MDT meeting). In such meetings, the UI provides information via three tabs, namely an Introduction Tab, Diagnosis Tab and a Treatment tab. Figures 6A-6C depict the UI at three respective MDT meetings with the Diagnosis tab selected (so that the information for patient diagnosis is displayed), such that Introduction and Treatment tabs remain selectable but are not fully displayed/expanded.

Specifically, Figure 6A is a screenshot of the UI in a first MDT meeting relating to a 'primary diagnosis and treatment selection' stage in the patient's care path. As detailed above, in Figure 6A, the Diagnosis tab of the UI is selected.

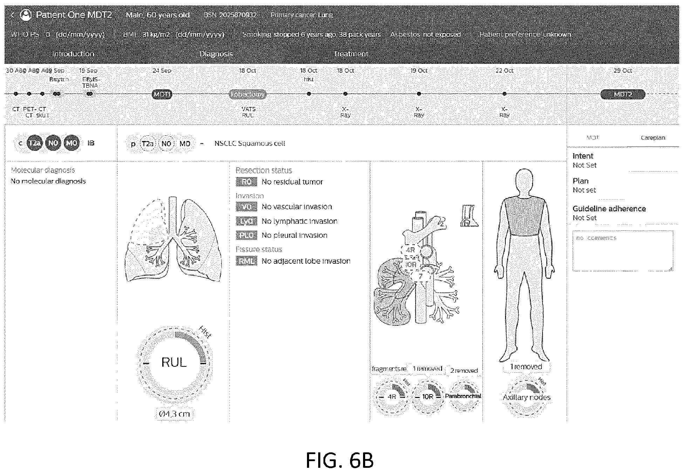

Figure 6B is a screenshot of the UI in a second MDT meeting relating to an 'evaluation after surgery' stage in the patient's care path. Again, as detailed above, in Figure 6B, the Diagnosis tab of the UI is selected.

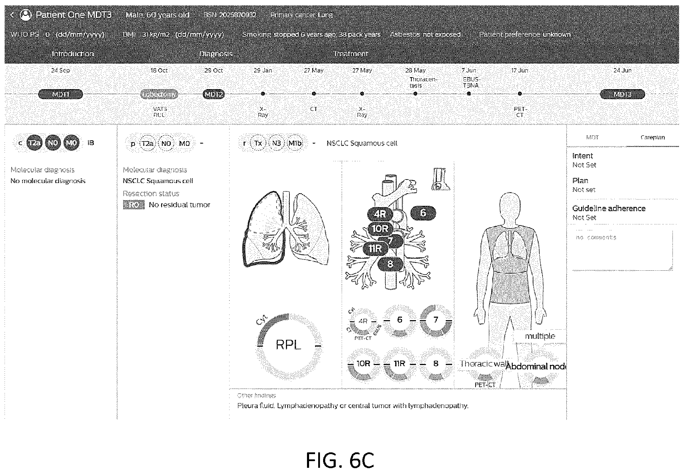

Figure 6C is a screenshot of the UI in a third MDT meeting relating to a 'recurrence' stage in the patient's care path. Again, as detailed above, in Figure 6C, the Diagnosis tab of the UI is selected.

From a comparison of the UI in each of Figures 6A-6C, it will be appreciated that the UI is driven by the point in the care path. For example, the displayed user-interface component information in the primary diagnosis phase (Figure 6A) differs from the information from the information displayed in the surgical evaluation phase (Figure 6B). In other words, the displayed user-interface component information is driven by the timeline of the patient, namely the timeline of the patient information can be considered as the subject-specific clinical information that will be used to determine the subset of the subject-specific clinical information. Said subset of the subject-specific clinical information will be used to determine the user-interface element. The timeline is also shown in the dashboard. This reflects the influence of the data weight driver; contextually relevant information is driven by patient data and clinical pathway data (i.e. the patient profile is matched to the position in the care path).

It is also noted that the UI is driven by discussion during the MDT meeting, wherein such discussions may cover an introduction phase, diagnosis phase and treatment selection. For example, the displayed information differs between the introduction tab, diagnosis tab and treatment selection tab. For instance, in the introduction screen, more weight may be given to the comorbidities than in the diagnosis screen. In other words, information of the discussion can be considered as clinical context information that will be used to determine the subset of the subject-specific clinical information. Said subset of the subject-specific clinical information can then be used to determine the user-interface element.

Embodiments may be configured to enable activation of dedicated UI modules (e.g. for diagnostic evaluation, for surgical evaluation, for treatment selection, etc.) depending on clinical context and/or subject-specific information. For instance, this could enable visualization of the result of dedicated prediction models to predict response to specific therapies (e.g. immunotherapy) and the various sources of diagnostic information (that describe the status of the immune system) on which a prediction is based, so as to predict a response.

From the above description, it will be understood that there is proposed a system that generates flexible and adaptive UIs for personalized decision support. Such a system may automatically generate the personalized user interfaces, which contain UI components and/or elements dedicated to a specific subject and context.

Embodiments may be used to provide the right data, in the right format and timing/ hierarchy in a clinical workflow and context (e.g., user role), and this may enable medical professionals to discuss a subject case based on a UI that is optimized to show the relevant data for that specific subject.

Embodiments may also utilize a UI engine to automatically generate (rather than handcraft) a UI that is optimized to show the abovementioned relevant subset of subject-specific information for a given problem at hand in a given context (e.g., user, physical space) using a set of UI components. The UI engine selects the optimal UI components and composes them into a dedicated UI for that particular subject, audience, clinical question, role, etc.

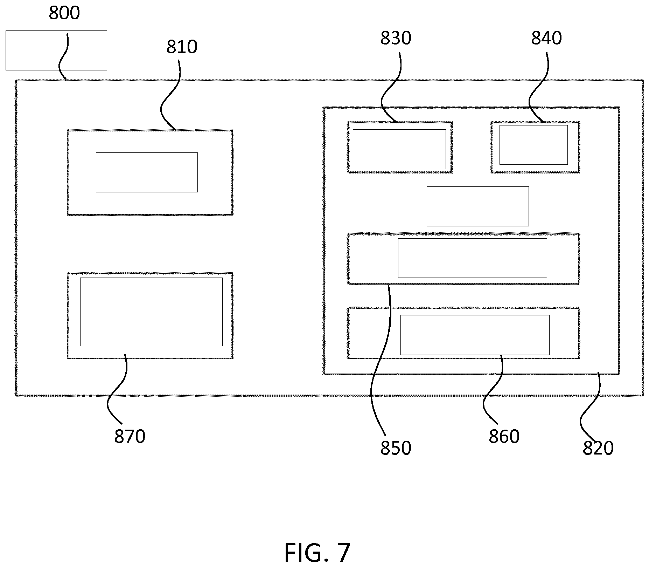

Figure 7 illustrates an example of a computer 800 within which one or more parts of an embodiment may be employed. Various operations discussed above may utilize the capabilities of the computer 800. For example, one or more parts of a system for providing a subject-specific user interface may be incorporated in any element, module, application, and/or component discussed herein.

The computer 800 includes, but is not limited to, PCs, workstations, laptops, PDAs, palm devices, servers, storages, and the like. Generally, in terms of hardware architecture, the computer 800 may include one or more processors 810, memory 820, and one or more I/O devices 870 that are communicatively coupled via a local interface (not shown).

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.).

The software in the memory 820 may include one or more separate programs, each of which comprises an ordered listing of executable instructions for implementing logical functions. The software in the memory 820 includes a suitable operating system (O/S) 850, compiler 840, source code 830, and one or more applications 860 in accordance with exemplary embodiments. As illustrated, the application 860 comprises numerous functional components for implementing the features and operations of the exemplary embodiments.

The operating system 850 controls the execution of other computer programs, and provides scheduling, input-output control, file and data management, memory management, and communication control and related services.

Application 860 may be a source program, executable program (object code), script, or any other entity comprising a set of instructions to be performed. When a source program, then the program is usually translated via a compiler (such as the compiler 840), assembler, interpreter, or the like, which may or may not be included within the memory 820, so as to operate properly in connection with the O/S 850.

The I/O devices 870 may include input devices such as, for example but not limited to, a mouse, keyboard, scanner, microphone, camera, etc. Finally, the I/O devices 870 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc.

If the computer 800 is a PC, workstation, intelligent device or the like, the software in the memory 820 may further include a basic input output system (BIOS) (omitted for simplicity).

When the computer 800 is in operation, the processor 810 is configured to execute software stored within the memory 820, to communicate data to and from the memory 820, and to generally control operations of the computer 800 pursuant to the software.

When the application 860 is implemented in software it should be noted that the application 860 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 860 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand various embodiments with various modifications are contemplated.

**Claims**

1. A method for generating a subject-specific user interface for Clinical Decision Support, the method comprising:

   obtaining clinical reference information regarding established clinical knowledge, the established clinical knowledge relating to a plurality of different clinical standards;
   obtaining subject-specific clinical information regarding a subject;
   determining, based on the subject-specific clinical information, clinical context information describing a relevance or importance of at least one of the plurality of different clinical standards;
   based on the clinical reference information, the subject-specific clinical information and the clinical context information, determining a subset of the subject-specific clinical information, the subset comprising subject-specific clinical information relevant to a clinical decision; and
   defining at least one of: a user-interface component; and a user-interface element based on the subset of the subject-specific clinical information.

2. The method of claim 1, wherein determining a subset of the subject-specific clinical information comprises:

associating weighting values to the subject-specific clinical information based on the clinical reference information and the clinical context information; and

identifying a subset of the subject-specific clinical information based on the weighting values associated with the subject-specific clinical information.

3. The method of claim 2, wherein associating weighting values to the subject-specific clinical information comprises:
processing the clinical reference information and the clinical context information with a heuristic algorithm to determine weighting values for the subject-specific clinical information.

4. The method of claim 2 or 3, wherein defining at least one of: a user-interface component; and a user-interface element based on the subset of the subject-specific clinical information comprises:

based on the weighting values associated with the subject-specific clinical information, identifying at least one data type of the subset of the subject-specific clinical information; and determining a user-interface component or user-interface element based on the identified at least one data type.

5. The method of claim 4, wherein determining a user-interface component or user-interface element based on the identified at least one data type comprises
associating component weighting values to the at least one data type; and
identifying a user-interface component or user-interface element from a set of available user-interface components and user-interface elements based on the component weighting values associated with the at least one data type.

6. The method of claim 4 or 5, wherein determining a user-interface component or user-interface element based on the identified at least one data type comprises:
identifying a user-interface component or user-interface element from a set of available user-interface components and user-interface elements, the identified user-interface component or user-interface element being configured to show data of the identified at least one data type.

7. The method of claim 4 or 5, wherein determining a user-interface component or user-interface element based on the identified at least one data type comprises:

determining if a set of available user-interface components and user-interface elements does not contain a user-interface component or user-interface element that is suitable for showing data of the identified at least one data type; and responsive to determining the set of available user-interface components and user-interface elements does not contain a user-interface component or user-interface element that is suitable for showing data of the identified at least one data type, defining a user-interface component or user-interface element for showing data of the identified at least one data type.

8. The method of any of claims 1 to 7, wherein obtaining clinical reference information comprises obtaining clinical reference information from at least one of:

a knowledge-based system comprising a clinical knowledge base with domain knowledge, rules and associations; and
a non-knowledge-based system comprising a learning component configured to generate clinical knowledge.

9. The method of any of claims 1 or 8, wherein the subject-specific clinical information comprises at least one of:

Subject data describing at least one physiological parameter or characteristic of the subject;
clinical data describing at least one medical parameter or characteristic of the subject; and
operational data describing at least one clinical test or procedure undertaken by the subject.

10. The method of any of claims 1 to 9, wherein the clinical reference information comprises a clinical pathway model,
and wherein determining clinical context information comprises:

analyzing the clinical pathway model of the subject-specific clinical information to identify a current location of the subject in a clinical pathway;
identifying one or more of the plurality of different clinical standards as being relevant or importance based on the identified current location of the subject in a clinical pathway;
determining a relevance or importance of the identified one or more clinical standards; and
determining clinical context information describing the determined relevance or importance of the identified one or more clinical standards.

11. The method of any of claims 1 to 10, wherein defining at least one of: a user-interface component; and a user-interface element comprises:

**EP 3 745 416 A1**

selecting a user-interface component or user-interface element from a library of user interface components and elements.

12. A computer program product for generating a subject-specific user interface for Clinical Decision Support, wherein the computer program product comprises a computer-readable storage medium having computer-readable program code embodied therewith, the computer-readable program code configured to perform all of the steps of any of claims 1 to 10.

13. A system for generating a subject-specific user interface for Clinical Decision Support, the system comprising:

an interface component configured to obtain clinical reference information regarding established clinical knowledge, the established clinical knowledge relating to a plurality of different clinical standards, and to obtain subject-specific clinical information regarding a subject;

an inference component configured to determine, based on the subject-specific clinical information, clinical context information describing a relevance or importance of at least one of the plurality of different clinical standards;

an analysis component configured to, based on the clinical reference information, the subject-specific clinical information and the clinical context information, determining a subset of the subject-specific clinical information, the subset comprising subject-specific clinical information relevant to a clinical decision; and

an output component configured to define at least one of: a user-interface component; and a user-interface element based on the subset of the subject-specific clinical information.

14. The system of claim 13, wherein the analysis component is configured to:

associate weighting values to the subject-specific clinical information based on the clinical reference information and the clinical context information; and

identify a subset of the subject-specific clinical information based on the weighting values associated with the subject-specific clinical information.

15. The system of claim 13 or 14, wherein the clinical reference information comprises a clinical pathway model,

and wherein the inference component is configured to:

analyze the clinical pathway model of the subject-specific clinical information to identify a current location of the subject in a clinical pathway;

identify one or more of the plurality of different clinical standards as being relevant or importance based on the identified current location of the subject in a clinical pathway;

determine a relevance or importance of the identified one or more clinical standards; and

determine clinical context information describing the determined relevance or importance of the identified one or more clinical standards.

100

105

110    120

125

110    130    120

135    140

150

FIG. 1

FIG. 2A

205

206

135

FIG. 2B

207

208

135

**FIG. 2C**

FIG. 3A

History

Weight = **4**

Staging

Weight = **12**

T

N

Detail
Weight = **1**

Detail
Weight = **2**

FIG. 3B

FIG. 3C

FIG. 4

FIG. 5

FIG. 6A

FIG. 6B

FIG. 6C

FIG. 7

Europäisches
Patentamt

European
Patent Office

Office européen
des brevets

# EUROPEAN SEARCH REPORT

Application Number

EP 19 17 7330

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/150183 A1 (SCHMITT MICHAEL D [US] ET AL) 11 June 2009 (2009-06-11)<br>* paragraphs [0029], [0037], [0084]; claim 1; figure 2 *<br>----- | 1-15 | INV.<br>G16H50/70<br>G16H10/60<br>G16H70/20 |

TECHNICAL FIELDS
SEARCHED      (IPC)

G16H

The present search report has been drawn up for all claims

| Place of search | Date of completion of the search | Examiner |
|---|---|---|
| Munich | 24 October 2019 | Beligny, Samuel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 17 7330

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2019

| Patent document cited in search report | Publication date | Patent family member(s) | Publication date |
|---|---|---|---|
| US 2009150183 A1 | 11-06-2009 | NONE | |

For more details about this annex : see Official Journal of the European Patent Office, No. 12/82